# EUROPEAN PATENT SPECIFICATION

(11) **EP 0 197 771 B1**
(45) Date of publication and mention of the grant of the patent: **30.06.1993**
(21) Application number: 86302479.0
(22) Date of filing: 03.04.1986
(51) Int. Cl.: A61F 13/00, A61F 13/10, A61F 7/03

(54) **Therapeutic pad**
Therapeutische Bandage
Bandage thérapeutique

(30) Priority: 04.04.1985 US 719986
(43) Date of publication of application: 15.10.1986
(60) Divisional application: 91111304.1
(73) Proprietor: LASTRAP INC, Mississauga Ontario L5L 1T1 (CA)
(72) Inventor: Last, Anthony J., Oakville Ontario L5L 1T1 (CA)
(74) Representative: Burford, Anthony Frederick

(56) References cited:
- EP-A- 0 099 010
- GB-A- 2 074 452
- US-A- 2 595 328
- US-A- 2 909 176
- US-A- 3 900 035

## Description

The present invention relates to a therapeutic pad, which may take the form of a vibration absorbing pad for use in connection with racquet and ball game playing or a passive heating pad for therapeutic application.

The increased playing of tennis, squash and racquet-ball by men and women over 30 years of age has led to the increase of a painful problem known as "tennis elbow". This condition usually occurs in people between 30 and 50 years of age, where muscles and tendons have become less supple and less able to absorb and dissipate vibrational shocks.

Epicondylalgia externa (tennis elbow) is not only found in racquet sports but can be aggravated by other pursuits, such as, golf and bowling. It can also be found in certain trades, such as carpentry, due to repeated hammering and driving of screws, and in house painting, due to the forward and backward stroke of the brush. Also, it can be found in industrial jobs involving pneumatic hammers and the like.

The motive power that enables the forearm to be rotated from side to side comes from the wrist and not the elbow. Therefore, the start of the problem is at the wrist during pronation and supination. Shock and/or vibration occurring at the wrist is propagated up the extensor muscles until it terminates in the outer and supporting parts of the attachment to the head of the humerus called the lateral epicondyle and also the extensor tendon that attaches the forearm extensor muscles to the epicondyle. The vibration can also be propagated up the flexor muscles in the same manner.

The forearm extensor muscles are those that come into play during the extension, raising or snapping of the wrist. Every time a tennis ball hits a racquet there is a certain force propagated up the forearm muscles which are already in tension due to the weight and acceleration of the racquet and the tension caused by the centrifugal force of the stroke. If the ball is mishit, an extra force is added which leads to an upward snap of the wrist. It is this extra stress that travels up the extensor muscles to the epicondyle and causes the trauma leading to inflammation. To some extent, aluminium metal or graphite racquets tend to reduce the stress because of their light weight and also racquets with oversized heads tend to reduce the problem because the larger area that the ball can hit, without twisting the racquet, reduces the number of mishits.

GB-A-2074452 discloses the control of epicondylalgia externa by a pad containing a highly viscous vibration-absorbing fluid maintained in close contact with the skin adjacent the extensor or flexor muscles. The pad may be maintained in position by means of an elastic tube or sleeve. An elastic strap, which can be tightened by Velcro(™) fasteners or the like around the widest part of the forearm, is provided to ensure close contact with the aforementioned muscles and the integrity of the device on the arm during violent exercise.

GB-A-2074452 states that the high viscosity fluid must be able to flow and can be liquid or semi-solid. Specific reference is made to the fluid being putty-like or composed of an uncured thermal setting glue or a silicone fluid. It has now been found advantageous to use a fluid having a viscosity of 100,000 to 1,000,000 Saybolt Universal Second (SUS) units at 100°F (38°C) and comprising a continuous liquid phase and finely-divided solid filler material dispersed therein.

Accordingly, in one aspect, the present invention provides a therapeutic device for absorbing vibration along body muscles tending to traumatize portions thereof, which includes a pad of highly-viscous, permanently-fluid vibration-absorbing material, characterized in that said material has a viscosity of 100,000 to 1,000,000 Saybolt Universal Second units at 100°F (38°C) and comprises a continuous liquid phase and finely-divided solid filler material dispersed therein.

In a preferred embodiment, the device is constructed for controlling epicondylalgia externa in a person to minimize the incidence thereof or relieve the symptoms thereof, by dimensioning the pad means to extend over an area of the forearm of a wearer of the device which is at least half of the area extensor or flexor muscles in the forearm projected to the forearm surface, and providing holding means for maintaining the pad means in contact with the forearm, thereby to dampen and absorb surface vibrations in the extensor and flexor muscles and minimize traumatization and consequent inflammation of the epicondyle of the forearm.

The device of this invention also may be used on other parts of the human frame where vibration can cause similar problems. An example is the occasional pain occurring in the lower leg, due to the vibration experienced by jogging on hard pavements.

Essential to the present invention is the use of a vibration-absorbing material of high viscosity in the range of 100,000 to 1,000,000 SUS units at 100°F (38°C). Vibration is essentially a pressure wave passing through a material. In liquids, adjacent molecules contact one another so as to propagate a wave motion through the liquid. If the molecules find it harder to move, then the vibrational energy is absorbed and degrades into heat through friction. High viscosity is one manner of inhibiting molecule motion and small solid particles alone aid in the absorption of energy. In the present invention, a vibration-absorbing material is employed in contact with body parts.

Preferably, the vibration-absorbing material is of low thermal conductivity. The material may also have a decreasing viscosity with increasing temperature.

In one preferred embodiment of the invention, the high viscosity absorbent material comprises a three-component system comprising a liquid polybutene, a petroleum wax and solid filler material. In this composition, the polybutene component acts as a high viscosity matrix material, and the petroleum wax acts as a skeleton around which the polybutene flows.

The filler material generates some heat by internal friction and increases viscosity. Suitable fillers are, for example, sawdust or powdered rubber.

Polybutene is available in a variety of grades and molecular weights from various manufacturers. A liquid polybutene having a number average molecular weight in the range of 1,000 to 1,500 is preferred. One such polybutene is that available from Ashland Chemicals under the trademark "Petrofin 300", which has a number average molecular weight of 1390. Polybutenes usually have a high viscosity at body heat temperatures but a much lower viscosity at higher values.

The presence of the petroleum wax and the solid filler material in the composition increases the stiffness of the mixture to provide a higher viscosity value, within the viscosity range of 100,000 to 1,000,000 SUS units at 100°F (38°C) (i.e. approximately body heat temperature).

The solid filler material may be a finely-divided inert solid, such as calcium carbonate, sawdust or powdered rubber.

When the material has a low thermal conductivity, the pad may also function as a passive heating pad for the therapeutic application of heat to a body muscle or group of muscles requiring treatment, for example, in the treatment of arthritis. While mainly useful for man, the passive heating pad may be used with other animals, such as horses.

In use, the passive heating pad is placed over the muscle, muscle group or joint where relief is required and is held there by a bandage, which may be a normal bandage or a tensor bandage. The skin below the pad commences to heat up, since the normal radiation of heat from the skin is inhibited by the pad. If the muscle or muscle group is exercised, the heat release is increased until perspiration commences.

The evaporation of the perspiration is inhibited as it is beneath the pad and the pad is in contact engagement with the skin and, therefore, the muscle or group below the pad increases in temperature. In order to maximize this effect, the pad moulds itself closely to the skin surface to inhibit the evaporation of the perspiration.

Increased heat in the muscles has the effect of increasing the blood flow, not only in the area under the pad, but also in adjacent areas. Increased blood flow has a salutary and healing effect on numerous minor injuries which can occur in the human frame, either at work or at play.

The present invention is described further, by way of illustration, with reference to the accompanying drawings, in which:
Figure 1 is a cut-away section of a forearm showing the extensor and flexor muscles and epicondyle;
Figure 2 is a drawing of one of the extensor muscles showing the muscle, ligament and epicondyle and the amplitude of surface vibrations occurring in the muscle;
Figure 3 is a perspective view of a device provided in accordance with a preferred embodiment of the invention; and
Figure 4 is a cross-sectional view of the vibration absorbing pad of the invention at the line A-A shown in Figure 3.

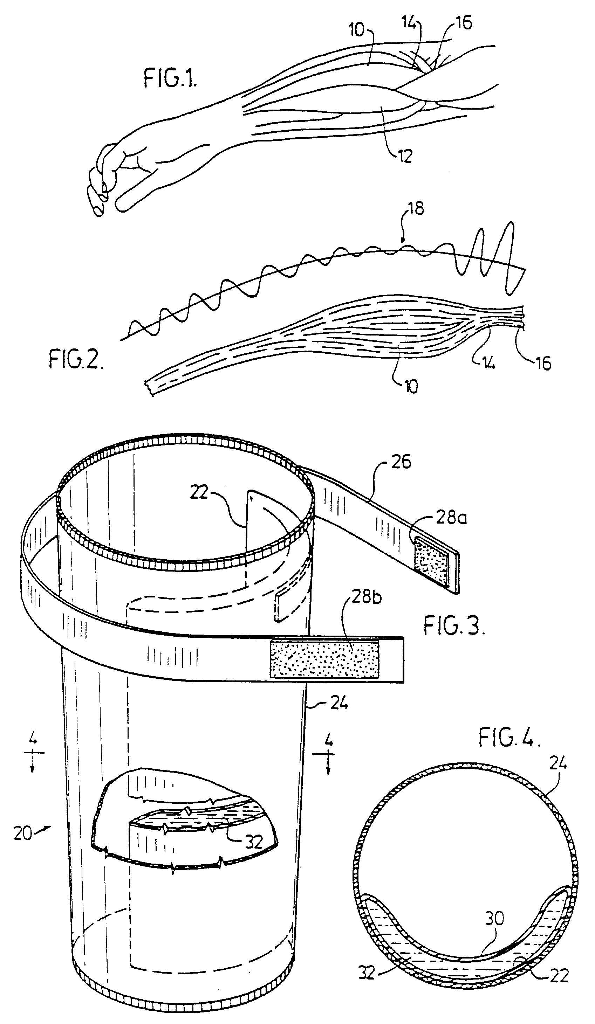

Referring now to the drawings, and in particular to Figures 1 and 2, wherein there is illustrated the problem to which the invention relates. In Figure 1, the numeral 10 designates the extensor group of muscles and numeral 12 the flexor group of muscles of a person's forearm. Numerals 14 and 16 designate the extensor tendon and epicondyle respectively of the extensor muscle.

In Figure 2, only one extensor muscle 10 is shown. As in Figure 1, numerals 14 and 16 designate the extensor tendon and epicondyle. Numeral 18 shows the amplitude of the surface vibrations as they leave the wrist area and proceed along the extensor muscles 10. They will be at their lowest amplitude at the largest cross-sectional area and, as the extensor muscle 10 necks down, so the vibration increases in amplitude to a maximum at the termination point, the epicondyle 16.

The propagation of the vibrations along the muscles is maintained until the muscle absorbs the energy and dissipates it throughout its length as heat. The muscle is itself shaped in such a way that any small vibration on its diameter rapidly increases in amplitude as the diameter becomes smaller. Those versed in the art of transmission line theory will recognize this fact and they will also recognize that, if conditions of resonance are present, the amplitudes of vibrations may become even higher. At the point where the muscle tendon 14 joins the epicondyle 16 there is an impedance change which becomes even greater at the attachment to the bone. This impedance change, which can be defined as a change in the ability to conduct or absorb vibrations, means that the energy must be released in the form of heat or strain on the epicondyle 16. It is the latter that causes the small surface tears and lesions leading to the inflammation of the epicondyle 16 and surrounding tissue.

In the present invention, the surface vibrations along the extensor muscles are absorbed and thereby reduces the trauma at the epicondyle 16. The application of a pad of viscous vibration-absorbing permanently-fluid material to the forearm increases the temperature in the muscle below the pad. The temperature increase is due to the inability of the perspiration at the skin surface to evaporate and to the friction and heat insulation of the pad itself. This increase in temperature is beneficial in that it leads to a greater absorption of vibrational energy. F. Dunn, in the Journal of the Acoustical Society of America, Volume 34, page 1545, in 1962, showed that the absorption coefficient of a vibration of 1 MHz in many types of biological tissue increases with increasing temperature.

Turning now to Figures 3 and 4, wherein there is illustrated a preferred embodiment of a vibration-absorbing device of the invention useful in the control of tennis elbow, a device 20 comprises a vibration absorbent pad 22 filled with viscous vibrating-absorbing permanently-fluid material, which is attached by means of a fastener 23 such as Velco (Trade Mark), to the inside of an elastic tube 24, which is shaped and sized to be fitted on the forearm of the user of the device 20 from the wrist to just below the joint of the elbow. The pad 22 extends for almost the whole length of the tube 24. The tube may be constructed of any convenient elastic material, such as, flexible woven or knitted elastic material, or a one-way stretch material which stretches circumferentially of the tube. Over the tube 24 at approximately the widest diameter of the forearm, an elastic strap 26 is attached at one end thereof (shown detached in the drawing) and surrounds the forearm and places extra tension on the pad 22 to prevent movement thereof during violent movement of the forearm on which the device 20 is worn. The strap 26 is fastened to itself by any suitable means such as Velcro(™) fasteners 28a and 28b or buttons.

As may be seen from the cross-sectional view of Figure 4, the pad 22 is a flat or slightly curved container 30, made from any suitable material, such as, heat-sealable nylon, conforming with the extensor (or flexor) muscle and extends from just above the wrist to just below the elbow. The container 30 is filled with a viscous material having a continuous liquid phase 32 which will absorb vibrations. the viscous liquid must be able to flow, so as to maintain contact with the muscles as they flex with forearm motion. The viscous liquid has a viscosity of 100,000 to 1,000,000 SUS units at 100°F (38°C) comprising a liquid polybutene having a number average molecular weight of 1,000 to 1,500. Preferably, the material has a low thermal conductivity of less than 0.1 W/(m.°C).

The device 20 may be positioned on a user's forearm with the pad 22 inside the forearm adjacent to the flexor muscles, or outside adjacent to the extensor muscles. Sufferers from tennis elbow may wear the pad during normal everyday activity. The pad 22 effectively absorbs small vibrations that tend to increase additively the inflammation of the epicondyle due to small actions, such as, lifting objects and event the action of writing. In this way, the inflamed area can get a more complete rest than is normally possible and this leads to a relief of the symptoms and pain of epicondylalgia externa. The device 20 should always be worn when heavy exercise of the arm is contemplated.

The invention is illustrated further by the following Example:
To test the suitability of materials for use as the vibration-absorbing liquid in this invention, comparative tests were performed to ascertain the ability of the materials to absorb high frequency sound waves. The sound wave pattern was considered equivalent to the wave form of the vibrations encountered along the extensor muscle.

Distilled and deionized water was used as a reference standard and its attenuation of 2.25 MHz pulses over a distance of 0.5 inch (1.3 cm) is essentially zero dB. The following Table I indicates the results obtained.

**Table I**

| Material | dB Attenuation | Rates of Attenuation |
|---|---|---|
| Water | 0 | 1:1 |
| Glycerine | 2 dB in | 1:1.25 |
| Soya Bean Oil | 2 dB out | 1.25:1 |
| Rubber composition ⁽¹⁾ | 42 dB out | 125:1 |
| LASTRAP formulation I ⁽²⁾ | 56 dB out | 630:1 |
| LASTRAP formulation II ⁽²⁾ | 76 dB out | 6300:1 |

| | | |
|---|---|---|
| Notes: (1) Hot melt adhesive HM 1500 - H.B. Fuller Co. | | |
| (2) Polybutene, petroleum wax and 35 wt.% CaCO₃ | | |
| (3) Polybutene, petroleum wax and 40 wt.% CaCO₃ | | |

The results were obtained in the following way. A piezo-electric receiver and transmitter of the 2.25 MHz pulses were placed opposite each other in a holder into which could be poured the material to be measured. The transmitter/receiver used to operate the transducers was a Branson Sonoray 600 series. This unit is an instrument using ultrasound for non-destructive testing and thickness gauging of solid materials and the 600 unit is equipped with attenuators in the receiver portion of the circuit. When the attenuation dB IN/OUT attenuators are in the OUT position, the maximum gain of the received signals from the test object is obtained. The gain (amplitude) of the received signals is decreased in fixed increments as each toggle switch is placed in the IN position. The total range provided by these controls is 62 dB, which permits comparison of the amplitudes of signals with ratios in excess of 1250 to 1.

Such comparisons were achieved in the test work by setting the signal from the reference material (water) at a convenient screen level (50% of the screen height) by adjustment of the GAIN control with all the attenuators in the IN position, which then became the "reference" amplitude signal.

For the unknown, the signal was increased in 2 dB steps by switching combinations of the dB controls to the OUT position until the amplitude of the unknown signal reaches the same amplitude as the reference signal. Of the materials tested, glycerine and soya bean oil had somewhat similar absorption characteristics to water while the uncured rubber and the LASTRAP formulations, all used in this invention, had significant vibration-absorbing capability.

In summary of this disclosure, the present invention relates to the relief of tennis elbow by the use of a novel device.

## Claims

1. A therapeutic device (20) for absorbing vibrations along body muscles tending to traumatize portions thereof, which includes a pad (22) of vibration-absorbing permanently-fluid material characterized in that said material has a viscosity of 100,000 to 1,000,000 Saybolt Universal Second units at 38°C (100°F) and comprises a continuous liquid phase and finely-divided solid filler material dispersed therein.

2. A device as claimed in Claim 1, wherein said material comprises liquid polybutene, a petroleum wax and said filler material.

3. A device as claimed in Claim 2, wherein the polybutene has a number average molecular weight of 1,000 to 1,500.

4. A device as claimed in Claim 3, wherein the polybutene has a number average molecular weight of 1,390.

5. A device as claimed in any one of Claims 2 to 4, wherein the solid filler material is calcium carbonate.

6. A device as claimed in any one of Claims 1 to 4, wherein the solid filler material is sawdust or powdered rubber.

7. A device as claimed in any one of the preceding claims, wherein the high viscosity material has a decreasing viscosity with increasing temperature.

8. A device as claimed in any one of the preceding claims, wherein the high viscosity material has a thermal conductivity of less than 0.1 W/(m.°C).

9. A device as claimed in any one of the preceding claims constructed for controlling epocondylalgia externa in a person to minimize the incidence thereof or relieve the symptoms thereof, wherein holding means (24-28) is provided for maintaining the pad (22) in contact with the forearm.

10. A device as claimed in Claim 9, wherein the holding means (24-28) comprises a forearm encircling tubular member (24) constructed of expandable material and the pad (22) is mounted to an internal surface of the tubular member (24).

11. A device as claimed in Claim 9 or Claim 10, wherein strap means (26) is attached to the tubular member (24) adjacent a wider diameter end thereof and encircling the tubular member (24) to secure the tubular member (24) to the largest diameter of the forearm.

12. A device as claimed in Claim 11, wherein tightening means (28) is associated with the free end of the strap means (26) for tightening the strap means (26) in tension about the tubular member (24) when positioned on a forearm.

## Patentansprüche

1. Therapeutische Vorrichtung (20) zur Absorbtion von Vibrationen entlang von Körpermuskeln, die dazu neigen, Abschnitte davon zu traumatisieren, die ein Kissen (22) aus vibrationsabsorbierendem permanent flüssigem Material umfaßt,
dadurch **gekennzeichnet,** daß
das Material eine Viskosität von 100.000 bis 1.000.000 Saybolt Universal Second Einheiten bei 38°C (100°F) hat, und eine kontinuierliche Flüssigphase sowie einen darin dispergierten fein verteilten festen Füllstoff aufweist.

2. Vorrichtung nach Anspruch 1, bei der das Material flüssiges Polybuten, ein Petroleumwachs und den Füllstoff umfaßt.

3. Vorrichtung nach Anspruch 2, bei der das Polybuten ein durchschnittliches Molekulargewicht von 1.000 bis 1.500 aufweist.

4. Vorrichtung nach Anspruch 3, bei der das Polybuten ein durchschnittliches Molekulargewicht von 1,390 aufweist.

5. Vorrichtung nach einem der Ansprüche 2 bis 4, bei der der feste Füllstoff Calciumcarbonat ist.

6. Vorrichtung nach einem der Ansprüche 1 bis 4, bei der der feste Füllstoff Sägemehl oder pulverisierter Gummi ist.

7. Vorrichtung nach einem der vorhergehenden Ansprüche, bei der das hochviskose Material eine abnehmende Viskosität bei ansteigender Temperatur aufweist.

8. Vorrichtung nach einem der vorhergehenden Ansprüche, bei der das hochviskose Material eine Wärmeleitfähigkeit von weniger als 0,1 W/(m.°C) aufweist.

9. Vorrichtung nach einem der vorhergehenden Ansprüche, gebaut, um Epocondylalgia externa in einer Person zu kontrollieren, um deren Auftreten zu minimieren oder ihre Symptome zu lindern, wobei ein Haltemittel (24-28) vorgesehen ist, um das Kissen (22) mit dem Unterarm in Berührung zu halten.

10. Vorrichtung nach Anspruch 9, bei das Haltemittel (24-28) ein den Unterarm umfassendens rohrförmiges Teil (24) aufweist, das aus expandierbarem Material gebaut ist, und das Kissen (22) an einer Innenfläche des rohrförmigen Teils (24) angeordnet ist.

11. Vorrichtung nach Anspruch 9 oder 10, bei der ein Gurtmittel (26) an dem Ende mit einem größerem Durchmesser des rohrförmigen Teils (24) angebracht ist, und das rohrförmige Teil (24) umgreift, um das rohrförmige Teil (24) an dem größten Durchmesser des Unterarms festzulegen.

12. Vorrichtung nach Anspruch 11, bei der ein Spannmittel (28) mit dem freien Ende des Gurtmittels (26) verbunden ist, um das Gurtmittel (26) um das rohrförmige Teil (24) in Spannung zu ziehen, wenn dieses an einem Unterarm angebracht ist.

## Revendications

1. Dispositif thérapeutique (20) pour absorber les vibrations le long des muscles du corps qui ont tendance à en traumatiser des parties, qui comprend un tampon (22) de matière fluide en permanence et absorbant les vibrations, caractérisé en ce que ladite matière a une viscosité d' 100 000 à 1 000 000 d'unités en secondes universelles de Saybolt à 38°C (100°F) et comprend une phase liquide continue et une matière de charge solide finement divisée dispersée à l'intérieur.

2. Dispositif selon la revendication 1, dans lequel ladite matière comprend du polybutène liquide, une cire de pétrole et ladite matière de charge.

3. Dispositif selon la revendication 2, dans lequel le polybutène a une masse moléculaire moyenne en nombre de 1 000 à 1 500.

4. Dispositif selon la revendication 3, dans lequel le polybutène a une masse moléculaire moyenne en nombre de 1 390.

5. Dispositif selon l'une quelconque des revendications 2 à 4, dans lequel la matière de charge solide est le carbonate de calcium.

6. Dispositif selon l'une quelconque des revendications 1 à 4, dans lequel la matière de charge solide est de la sciure ou du caoutchouc pulvérulent.

7. Dispositif selon l'une quelconque des revendications précédentes, dans lequel la viscosité de la matière à haute viscosité décroît lorsque la température croît.

8. Dispositif selon l'une quelconque des revendications précédentes, dans lequel la matière à haute viscosité a une conductivité thermique inférieure à 0,1 W(m.°C).

9. Dispositif selon l'une quelconque des revendications précédentes, construit pour maîtriser l'épicondylalgie externe chez une personne pour minimiser sont incidence ou faire disparaître ses symptômes, dans lequel des dispositifs de maintien (24-28) sont prévus pour maintenir le tampon (22) en contact avec l'avant-bras.

10. Dispositif selon la revendication 9, dans lequel les dispositifs de maintien (24-28) comprennent un élément tubulaire (24) entourant l'avant-bras, construit en matière expansible, et le tampon (22) est monté sur une surface interne de l'élément tubulaire (24).

11. Dispositif selon la revendication 9 ou la revendication 10, dans lequel une sangle (26) est fixée à l'élément tubulaire (24) en position adjacente à une extrémité de plus grand diamètre de celui-ci et en encerclant l'élément tubulaire (24) pour fixer l'élément tubulaire (24) sur le plus grand diamètre de l'avant-bras.

12. Dispositif selon la revendication 11, dans lequel un dispositif de serrage (28) est associé d l'extrémité libre de la sangle (26) pour serrer la sangle (26) en tension autour de l'élément tubulaire (24) lorsqu'il est placé sur un avant-bras.
